# EUROPEAN PATENT SPECIFICATION

(11) **EP 3 331 546 B1**
(45) Date of publication and mention of the grant of the patent: **04.10.2023**
(21) Application number: 16751668.1
(22) Date of filing: 02.08.2016
(51) Int. Cl.: A61K 38/12, A61K 31/497, A61P 35/00, A61P 25/28, G01N 33/566, A61K 38/19, C12Q 1/68, A61K 38/16

(54) **CXCR4 INHIBITOR FOR THE TREATMENT OF CANCER**
CXCR4-ANTAGONIST ZUR BEHANDLUNG VON KREBS
ANTAGONISTE DE CXCR4 POUR LE TRAITEMENT DU CANCER

(30) Priority: 03.08.2015 US 201562200177 P
(43) Date of publication of application: 13.06.2018
(73) Proprietor: Biokine Therapeutics Ltd., 7403617 Nes Ziona (IL); BIOLINERX LTD., 7177871 Modiln (IL)
(72) Inventor: PELED, Amnon, 6777634 Tel-Aviv (IL); PEREG, Yaron, 6085000 Shoham (IL); ABRAHAM, Michal, 9077243 Mevasseret Zion (IL); KLEIN SILBERMAN, Shiri, 7173310 ModiIn (IL)
(74) Representative: van Breda, Jacobus
(86) International application number: PCT/IL2016/050845
(87) International publication number: WO 2017/021963

(56) References cited:
- WO-A1-2015/063768
- WO-A2-2012/058241
- ALEXEY V DANILOV ET AL: "Toward a cure for chronic lymphocytic leukemia: an attack on multiple fronts", EXPERT REVIEW OF ANTICANCER THERAPY, vol. 13, no. 9, 1 September 2013 (2013-09-01), pages 1009-1012, XP055307767, GB ISSN: 1473-7140, DOI: 10.1586/14737140.2013.825424 cited in the application
- SUNG HONG ET AL: "Inhibition of tumor growth and histopathological changes following treatment with a chemokine receptor CXCR4 antagonist in a prostate cancer xenograft model", ONCOLOGY LETTERS, vol. 72, no. 1, 6 August 2013 (2013-08-06) , pages 122-1323, XP055307591, GR ISSN: 1792-1074, DOI: 10.3892/ol.2013.1515
- DAO-FENG WANG ET AL: "Effects of CXCR4 gene silencing by lentivirus shRNA on proliferation of the EC9706 human esophageal carcinoma cell line", TUMOR BIOLOGY, vol. 34, no. 5, 7 June 2013 (2013-06-07), pages 2951-2959, XP055307592, CH ISSN: 1010-4283, DOI: 10.1007/s13277-013-0858-0
- QI WANG ET AL: "Stromal cell-derived factor 1 decreases -amyloid deposition in Alzheimer's disease mouse model", BRAIN RESEARCH, ELSEVIER, AMSTERDAM, NL, vol. 1459, 7 April 2012 (2012-04-07), pages 15-26, XP028489968, ISSN: 0006-8993, DOI: 10.1016/J.BRAINRES.2012.04.011 [retrieved on 2012-04-17]
- R. E. DONAHUE ET AL: "Plerixafor (AMD3100) and granulocyte colony-stimulating factor (G-CSF) mobilize different CD34+ cell populations based on global gene and microRNA expression signatures", BLOOD, vol. 114, no. 12, 17 September 2009 (2009-09-17), pages 2530-2541, US ISSN: 0006-4971, DOI: 10.1182/blood-2009-04-214403

## Description

### FIELD AND BACKGROUND OF THE INVENTION

The present invention, in some embodiments thereof, relates CXCR4 binding agents that up-regulate miR-15a and miR-16-1, and uses thereof, as expressed in the claims.

CXCR4 is a G-protein coupled receptor that mediates the activity of CXC chemokines. To date, its only identified cognate ligand is CXCL12, also known as Stromal Cell-Derived Factor-1 (SDF-1). CXCR4 plays an important role in mammalian development, mediating the migration and motility of tissue and hematopoietic stem and progenitor cells. Both CXCR4 and SDF-1 knock-out mice show embryonic lethality with essentially identical phenotypes involving tissue and vascular malformations, supporting the hypothesis that CXCR4 is the key receptor for the activity of SDF-1 (CXCR7 is a second known receptor of SDF-1). CXCR4 continues to be broadly expressed in the adult, with high levels detected on bone marrow stem and progenitor cells, various circulating lymphocytes (B-cells, activated T-cells), as well as endothelial precursor cells, and tissue macrophages and fibroblasts.

CXCR4 is likely to play a pleiotropic role in human cancer. Its expression is upregulated in many tumor types, including cancers of the breast, lung, colon, pancreas, brain, prostate, ovary, as well as hematopoietic cancers. Some literature reports suggest that SDF-1 may act through CXCR4 as a growth and/or survival factor for some tumors. In models of metastatic cancer, CXCR4 positive tumors were shown to metastasize to distant sites, and this activity was inhibited by agents that silence the CXCR4 gene or antibodies that block CXCR4 or SDF-1. Consistent with this view, many common sites of metastasis in human cancer, such as bone marrow, lung, lymph node, and liver, express high levels of SDF-1. CXCR4 is expressed on stem cell-like or tumor initiating subpopulations of many tumors, and may mediate the ability of these cells to support the recurrence and metastatic spread of cancers. Furthermore, CXCR4 is expressed on endothelial precursor cells (EPCs), and its activity is required for incorporation of EPCs into functional vessels during angiogenesis. This may make a significant contribution to the vascularization and survival of tumors. CXCR4 signaling can also lead to induction of pro-angiogenic cytokines (e.g., VEGF), as well as integrins, adhesion molecules and matrix degrading enzymes that may mediate invasion by tumor cells. Lastly, CXCR4 expression is detected on tumor infiltrating lymphocytes and fibroblasts, as well as tumor associated macrophages. These cells tend to suppress immune recognition and attack on the tumor, and remodel the tumor microenvironment to encourage tumor growth and metastasis.

The multiple roles of CXCR4 in tumor growth, vascularization, and metastasis, and its broad expression in many common tumor types, make this receptor an attractive target for therapeutic intervention using inhibitory agents.

4F-benzoyl-TN14003 (also known as BKT140, hereinafter BL-8040), is a 14-residue bio stable synthetic peptide of SEQ ID NO:14 developed as a specific CXCR4 antagonist. It has been shown that BL-8040 binds the CXCR4 receptor with high affinity and long receptor occupancy. BL-8040 was found to be toxic against several tumors such as myeloid leukemia, hematopoietic tumors and non-small cell lung cancer (International Patent Application No. IL2014/050939 and International Patent Application Publication No. WO2013/16089556053 and WO2008/075370).

US Patent Application No. 20070238115 teaches use of CXCL12 for treatment of carcinoma.

US Patent Application No. 20130281423 teaches the use of CXCL12 inhibitors for reducing the resistance of a tumor cell to a chemotherapeutic agent.

Singh et al., Mol Cancer Ther January 2009 8; 178-184 teaches gp120-IIIB for the treatment of prostate cancer.

Danilov, A. V., et al., (2013). Expert Review of Anticancer Therapy, 13(9), 1009-1012 teaches the use of plerixafor in combination with BC1-2 inhibitors for the treatment of chronic lymphocytic leukemia (CLL).

### SUMMARY OF THE INVENTION

According to an aspect of some embodiments of the present invention there is provided a therapeutically effective amount of SEQ ID NO: 14 for use in rendering a BC1-2 antagonist resistant subject more susceptible to treatment with a BC1-2 antagonist, wherein said subject is suffering from cancer and wherein SEQ ID NO: 14 increases the expression of miR-15a in the cells of said cancer and wherein said BCL-2 antagonist is selected from the group consisting of Oblimersen, SPC-2996, RTA-402, Gossypol, AT-101, Obatoclax mesylate, A-371191, A-385358, A-438744, ABT-737, ABT-199, AT-101, BL-11, BL-193, GX-15-003, 2-Methoxyantimycin A3, HA-14-1, KF-67544, Purpurogallin, TP-TW-37, YC-137 and Z-24.

According to some embodiments of the invention, the BCl-2 antagonist is ABT-199.

According to some embodiments of the invention, the cancer is selected from the group consisting of a hematological cancer, neuroblastoma, retinoblastoma, bladder cancer, esophageal carcinoma, sarcomas, colorectal cancer, melanoma, parathyroid adenoma, and breast cancer.

According to some embodiments of the invention, the hematological cancer is selected from the group consisting of hematological malignancy is selected from the group consisting of Chronic Myelogenous Leukemia (CML), CML accelerated phase, or blast crisis, multiple myeloma, Hypereosinophilic Syndrome (HES), myelodysplastic syndrome (MDS), acute lymphocytic leukemia (ALL), acute myeloid leukemia (AML), acute promyelocytic leukemia (APL), chronic lymphocytic leukemia (CLL), chronic neutrophilic leukemia (CNL), acute undifferentiated leukemia (AUL), anaplastic large-cell lymphoma (ALCL), prolymphocytic leukemia (PML), juvenile myelomonocytic leukemia (JMML), adult T-cell ALL, AML with trilineage myelodysplasia (AML/TMDS), mixed lineage leukemia (MLL), myeloproliferative disorders (MPD), multiple myeloma, (MM) and myeloid sarcoma.

According to some embodiments of the invention, the cancer is a hematological cancer.

### BRIEF DESCRIPTION OF THE SEVERAL VIEWS OF THE DRAWINGS

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:

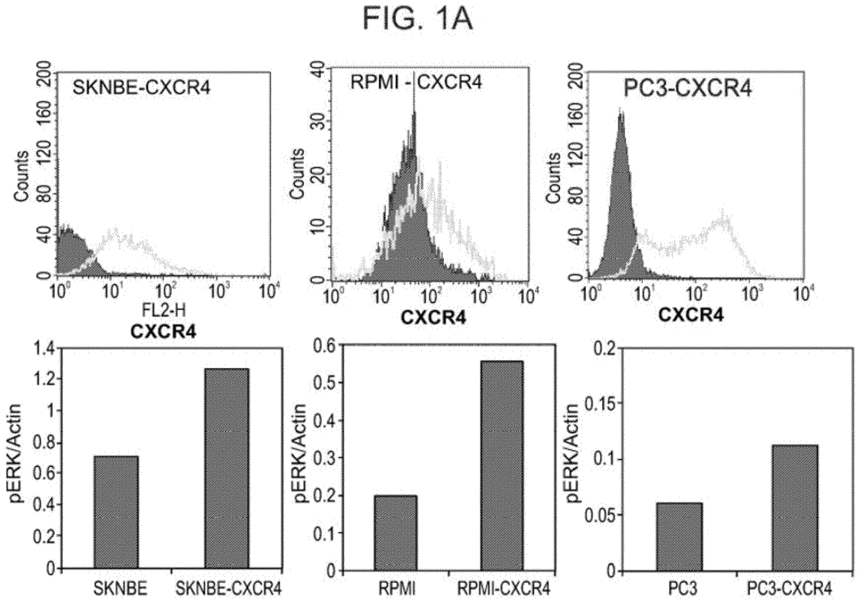

FIG. 1A illustrates that over-expression of CXCR4 increases the level of pERK. Cells (SKNBE, RPMI and PC3 cells) were stained with PE-conjugated anti-human CXCR4 clone 12G5. Expression of CXCR4 was measured on SKNBE, RPMI and PC3 cells (dark grey) and in cells genetically modified to over-express CXCR4 (light grey line) by FACS analysis. Over-expression of CXCR4 induced an elevation in the level of pERK in SKNBE, PC3 and RPMI cells as demonstrated by Western Blot. Cells were cultured with 1% FCS RPMI medium for 24 hr. Whole-cell lysates were assayed by Western blots and levels of pERK were detected and normalized to β-actin to ensure equal protein loading.

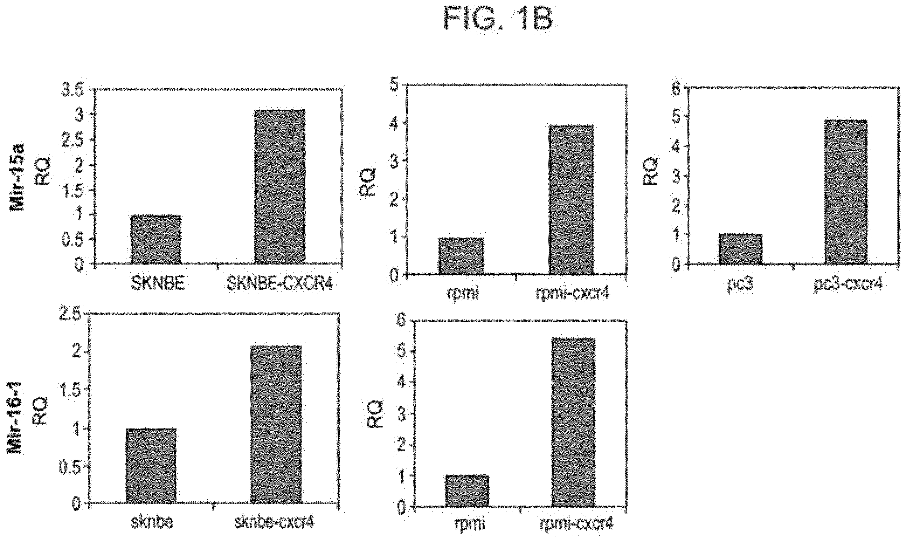

FIG. 1B illustrates that over-expression of CXCR4 up-regulates the expression of miR-15a and miR-16-1. Expression of miR-15a and miR-16-1 was assessed by RT-PCR in SKNBE, RPMI and PC3 cells following over-expression of CXCR4.

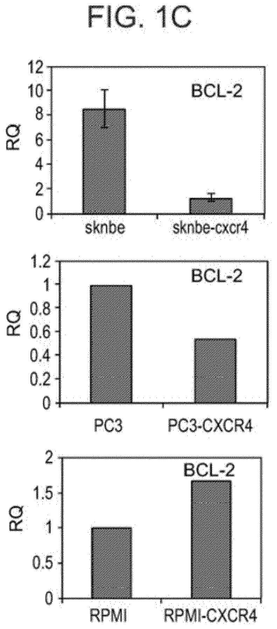

FIG. 1C illustrates that over-expression of CXCR4 down-regulates the mRNA expression of miR-15a and miR-16-1 target gene BCL-2 as analyzed by RT-PCR. In SKNBE cells, over-expression of CXCR4 down-regulated the expression of BCL-2 mRNA. Over-expression of CXCR4 down-regulated the expression of BCL-2 mRNA in PC3 cells. In RPMI cells, over-expression of CXCR4 did not down-regulate the expression of BCL-2.

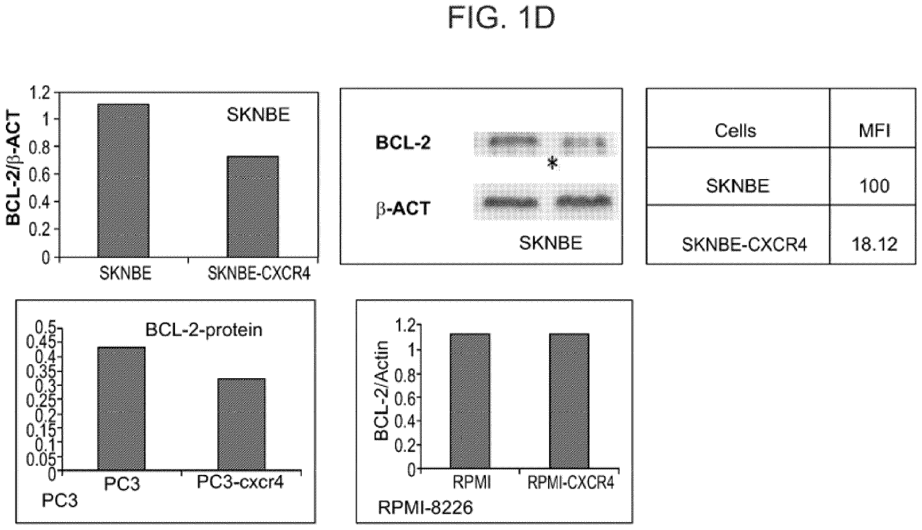

FIG. 1D illustrates that over-expression of CXCR4 down-regulates BCL-2 protein expression in SKNBE cells, as analyzed by Western Blot (WB) and by FACS analysis. Whole-cell lysates were assayed by Western blots and levels of BCL-2 were detected and normalized to the β-actin detection to confirm equal protein loading. Mean fluorescence intensity (MFI) of BCL-2 was evaluated by FACS following intracellular staining of anti-Human BCL-2. BCL-2 protein was down-regulated following over-expression of CXCR4 in PC3 cells but did not change in RPMI-8226 cells as demonstrated by Western Blot.

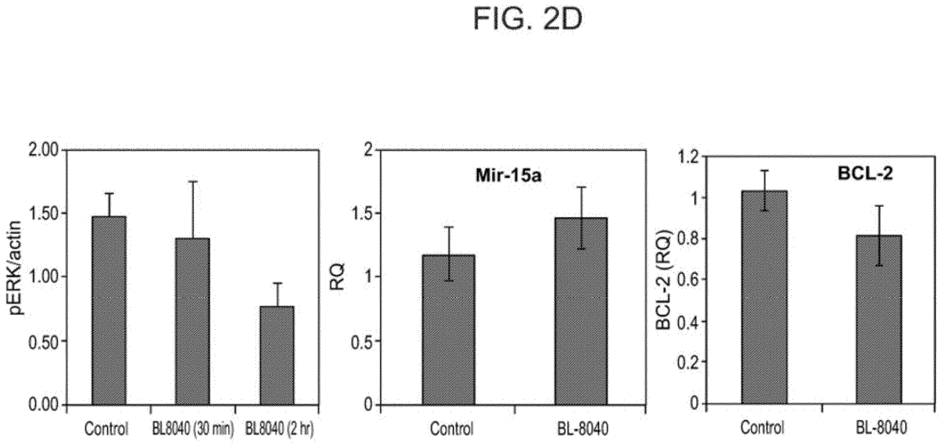

FIG. 2D illustrates that treatment of SKNBE with the CXCR4 binding agent, BL8040, inhibits ERK signaling, inducing miR-15a expression and reducing BCL-2 expression levels. Analysis of expression of pERK was performed by Western Blot (WB). SKNBE cells were cultured with 1% FCS RPMI medium for 24 hr after which BL-8040 (20uM) was added to the cells for 30 minutes or 2 hr. Whole-cell lysates were assayed by WB and levels of pERK were detected and normalized to β-actin to confirm equal protein loading. The level of miR-15a and BCL-2 was assessed by RT-PCR in SKNBE cells following a 24 hr incubation in the presence of BL-8040 (20uM) with 10% FCS medium.

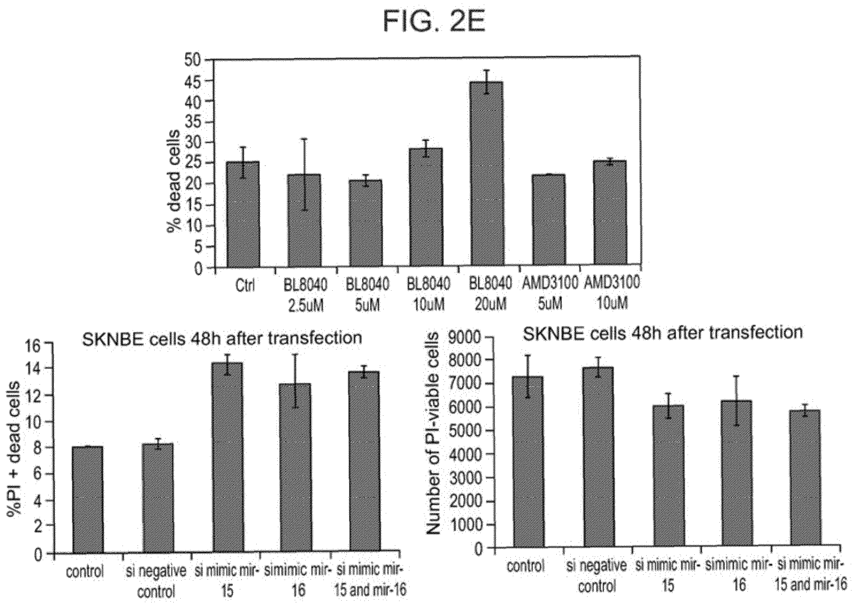

FIG. 2E illustrates that up-regulation of miR-15a by transfection of SKNBE cells with miR-15a or miR-16-1 induced cell death. The direct effect of miR-15a and mir-16-1on survival and viability of cells was analyzed following transfection with miR-15a and mirR-16-1to SKNBE cells. 48 hr following transfection, viability of cells was assessed by FACS following staining with PI.

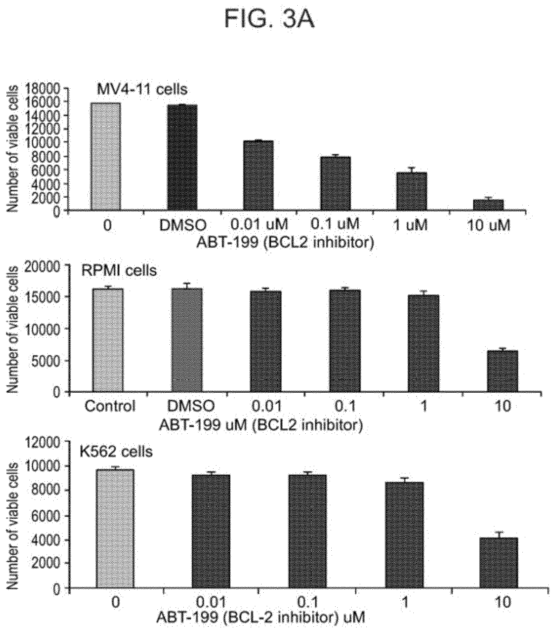

FIG. 3A illustrates that MV-V4-11 cells are dependent on BCL-2 for their survival in vitro and can be induced to undergo apoptosis using ABT-199. MV4-11, RPMI and K562 cells were incubated for 24 hr in 1% FCS RPMI medium in the presence of the BCL-2 inhibitor ABT-199 (0.01, 0.1, 1 and 10 uM). Viability of cells was evaluated using FACS analysis following PI staining.

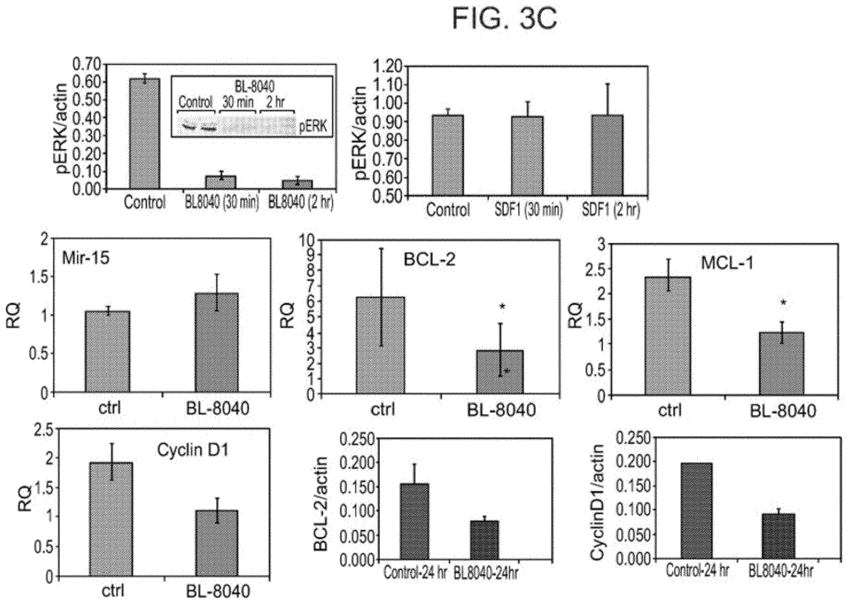

FIG. 3C illustrates that the CXCR4 binding agent, BL8040, induces rapid and dose dependent apoptosis of MV4-11 cells, inhibits ERK signaling and induces miR-15a expression and significantly reduces BCL2 expression levels. MV4-11 cells were cultured with 1% FCS RPMI medium for 24 hr. 24 hr after incubation, BL-8040 (8uM) was added to the cells for 30 minutes or 2 hr. Whole-cell lysates were assayed by WB and levels of pERK were detected and normalized to the β-actin to confirm equal protein loading. MV4-11 cells were incubated for 24 hr in the presence of BL-8040 (8uM) with 1% FCS medium. Level of miR-15a, mRNA of BCL-2 was assessed by RT-PCR and the level of BCL-2 protein was evaluated by WB.

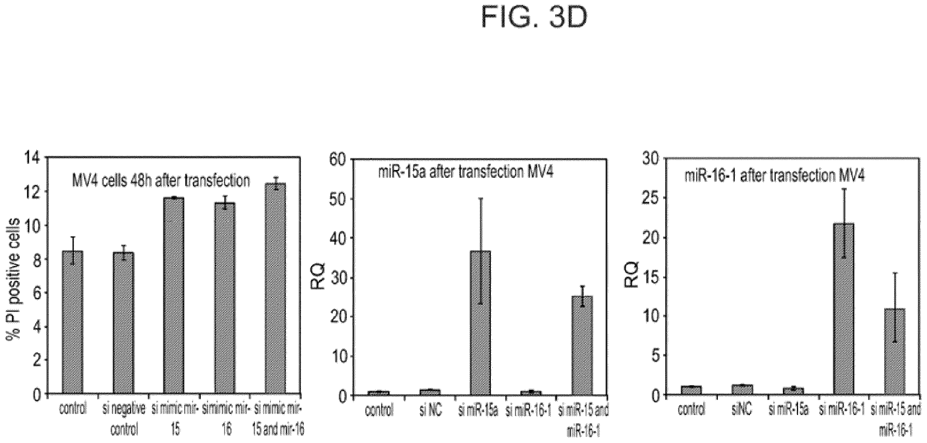

FIG. 3D illustrates that transfection of MV4-11 cells with miR-15a or miR-16-1 induces cell death. Analysis of the direct effect of miR-15a and mir-16-1 on survival and viability of MV4-11 cells was performed following transfection of miR-15a and miR-16-1to the cells. 48 hr following transfection, viability of the cells was assessed by FACS following staining with PI. Level of miR-15a and miR-16-1-1 was measured by RT-PCR 24 hr post transfection.

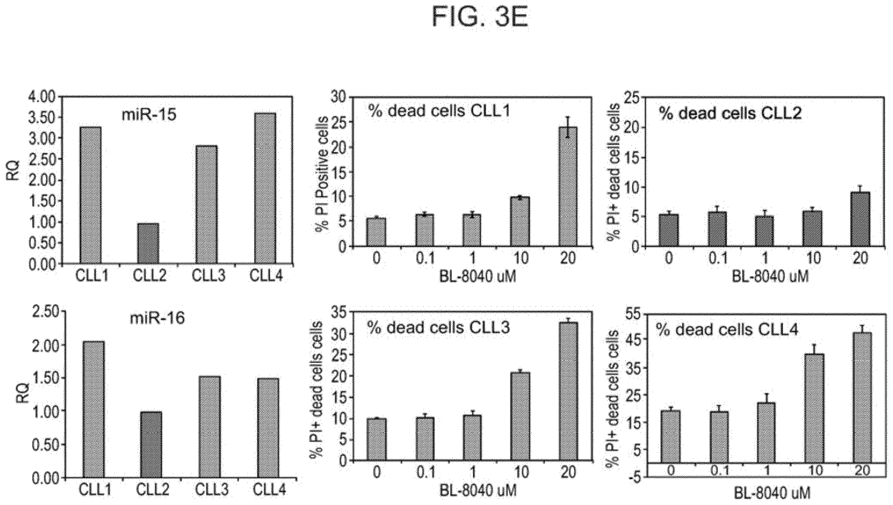

FIG. 3E illustrates that BL8040 was unable to induce cell death in CLL cells that do not express miR-15a/16-1. Level of miR-15a was evaluated in cells of CLL patients using RT-PCR. The ability of BL-8040 to kill cells from CLL patients was measured following incubation of cells for 24 hr at 1% FCS medium in the presence of BL-8040 (0.1, 1, 10 and 20 uM). Viability of cells was assessed by FACS following staining with PI.

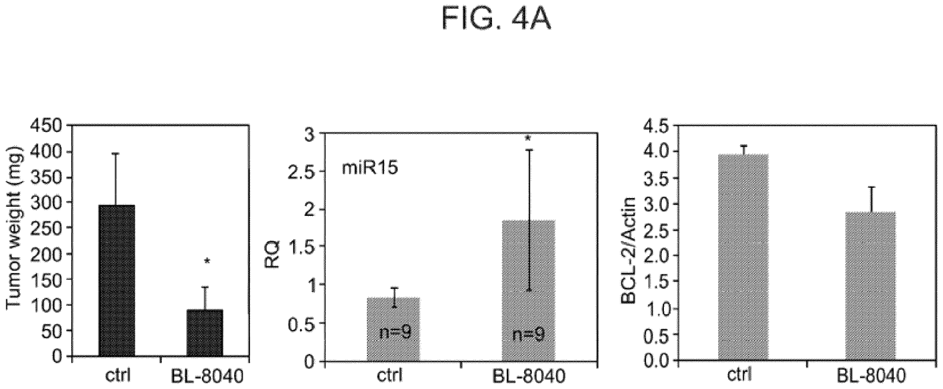

FIG. 4A illustrates that in-vivo treatment of BL8040 significantly increases miR-15a and down regulated BCL2 in a neuroblastoma mice model. NSG mice were injected with SKNBE cells into the left adrenal. One week later BL-8040 was SC injected daily for 14 days at a concentration of 400 µg/mouse. 24 hr after the last BL-8040 injection, animals were sacrificed; tumors were harvested, measured and weighed. RNA and total protein were extracted from the tumor and the level of miR-15a was evaluated by qRT-PCR. The level of BCL-2 protein was evaluated by Western blot.

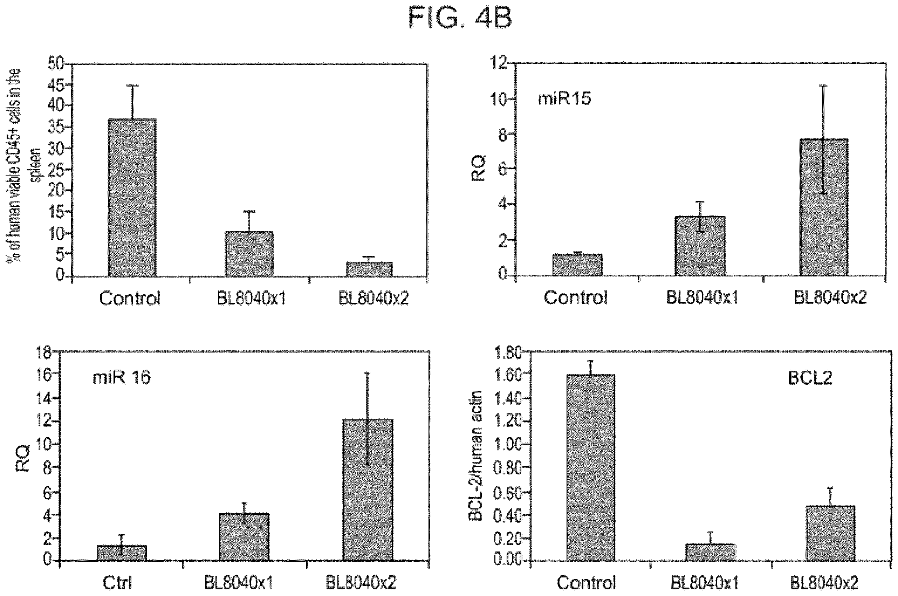

FIG. 4B illustrates that in the in-vivo AML model of MV4-11 cells, reduction in AML cells in the spleen is associated with a significant increase in miR-15a/16-1 and down regulation of BCL2 in human cells. NOD SCID gamma (NSG) mice were engrafted with MV4-11 cells. Engraftment of AML cells was allowed for 3 weeks following transplantation. At day 21, BL-8040 was SC injected into mice at 400µg/mouse once daily for one or two days (BL8040 x1, BL8040x2, respectively). 24 hr after the last injection, the mice were sacrificed and the level of human CD45+ AML cells in the spleen was evaluated by FACS. RNA was extracted from the spleen and the level of miR-15a, Mir-16-1was evaluated by qRT-PCR. The level of BCL-2 protein was evaluated by Western blot.

### DESCRIPTION OF SPECIFIC EMBODIMENTS OF THE INVENTION

CXCR4 is over-expressed in the majority of tumor cells and its expression typically correlates with bad prognosis. The present inventors have now found that over-expression of CXCR4 on tumor cells and stimulation of cells with CXCL12 leads to up-regulation of miR-15a/16-1 and consequently down regulation of their target gene BCL-2. Furthermore, overexpression of CXCR4 in these cells increases tumorigenesis and shifts their oncogenic dependency from BCL2 to CXCR4. The present inventors further showed that the CXCR4 binding agent BL8040 has a similar effect on miR-15a/16-1. Moreover, BL8040 inhibits ERK signaling (see Fig. 2D and 3C).

Thus, BL8040 induced apoptosis of tumor cell by inhibiting survival signals while keeping apoptotic gene expression low both in vitro and in vivo. The present results suggest that overexpression of CXCR4 may override the survival dependency of tumor cells on BCL-2 leading to resistance of tumor cells to inhibition of these pathways.

Thus, according to a first aspect of the present invention there is provided a therapeutically effective amount of SEQ ID NO: 14 for use in rendering a BC1-2 antagonist resistant subject more susceptible to treatment with a BC1-2 antagonist, wherein said subject is suffering from cancer and wherein SEQ ID NO: 14 increases the expression of miR-15a in the cells of said cancer and wherein said BCL-2 antagonist is selected from the group consisting of Oblimersen, SPC-2996, RTA-402, Gossypol, AT-101, Obatoclax mesylate, A-371191, A-385358, A-438744, ABT-737, ABT-199, AT-101, BL-11, BL-193, GX-15-003, 2-Methoxyantimycin A3, HA-14-1, KF-67544, Purpurogallin, TP-TW-37, YC-137 and Z-24.

As used herein, the term cancer refers to proliferative diseases including carcinoma, lymphoma, blastoma, sarcoma, and leukemia. The cancer may for example be a solid tumors Benign Meningioma, Mixed tumors of salivary gland, Colonic adenomas; Adenocarcinomas, such as Small cell lung cancer, Kidney, Uterus, Prostate, Bladder, Ovary, Colon, Sarcomas, Liposarcoma, myxoid, Synovial sarcoma, Rhabdomyosarcoma (alveolar), Extraskeletel myxoid chondrosarcoma, Ewing's tumor; other include Testicular and ovarian dysgerminoma, Retinoblastoma, Wilms' tumor, Neuroblastoma, Malignant melanoma, Mesothelioma, breast, skin, prostate, and ovarian.

According to a particular embodiment, the cancer is neuroblastoma, retinoblastoma bladder cancer, esophageal carcinoma, sarcomas, colorectal cancer, melanoma, parathyroid adenoma, and breast cancer.

According to another embodiment, the cancer is a solid tumor.

According to yet another embodiment, the cancer is hematological malignancy.

The term "hematological malignancy" herein includes a lymphoma, leukemia, myeloma or a lymphoid malignancy, as well as a cancer of the spleen and the lymph nodes. Exemplary lymphomas that are amenable to treatment with the disclosed agents include both B cell lymphomas and T cell lymphomas. B-cell lymphomas include both Hodgkin's lymphomas and most non-Hodgkins lymphomas. Non-limiting examples of B cell lymphomas include diffuse large B-cell lymphoma (DLBCL), follicular lymphoma (FL), mucosa-associated lymphatic tissue lymphoma (MALT), small cell lymphocytic lymphoma (overlaps with chronic lymphocytic leukemia), mantle cell lymphoma (MCL), Burkitt's lymphoma, mediastinal large B cell lymphoma, Waldenstrom macroglobulinemia, nodal marginal zone B cell lymphoma (NMZL), splenic marginal zone lymphoma (SMZL), intravascular large B-cell lymphoma, primary effusion lymphoma, lymphomatoid granulomatosis. Non-limiting examples of T cell lymphomas include extranodal T cell lymphoma, cutaneous T cell lymphomas, anaplastic large cell lymphoma, and angioimmunoblastic T cell lymphoma. Hematological malignancies also include leukemia, such as, but not limited to, secondary leukemia, acute myelogenous leukemia (AML; also called acute lymphoid leukemia), chronic myelogenous leukemia (CML), B-cell prolymphocytic leukemia (B-PLL), acute lymphoblastic leukemia (ALL) and myelodysplasia (MDS). Hematological malignancies further include myelomas, such as, but not limited to, multiple myeloma (MM), smoldering multiple myeloma (SMM) and B-cell chronic lymphocytic leukemia (CLL).

According to a particular embodiment, the hematological malignancy is chronic myelogenous leukemia (CML). The term CML includes imatinib-resistant CML, CML tolerant to second/third generation Bcr-Abl TKIs (e.g., dasatinib and nilotinib), imatinib-intolerant CML, accelerated CML, and lymphoid blast phase CML.

Other hematological and/or B cell- or T-cell-associated cancers are encompassed by the term hematological malignancy. For example, hematological malignancies also include cancers of additional hematopoietic cells, including dendritic cells, platelets, erythrocytes, natural killer cells, and polymorphonuclear leukocytes, e.g., basophils, eosinophils, neutrophils and monocytes. It should be clear to those of skill in the art that these pre-malignancies and malignancies will often have different names due to changing systems of classification, and that patients having lymphomas classified under different names may also benefit from the therapeutic regimens of the present invention.

Subjects treated according to this aspect of the present invention are typically mammalian subjects, e.g., humans.

The term "Bcl-2" as used herein refers to the Bcl-2 protein (Swiss Prot ID No. P10415), a member of the Bcl-2 family of proteins (Cory, S., and Adams, J. M., Nature Reviews Cancer 2 (2002) 647-656; Adams, Genes and Development 17 (2003) 2481-2495; Danial, N. N., and Korsmeyer, S. J., Cell 116 (2004) 205-219; Petros, A. M., Biochim Biophys Acta 1644 (2004) 83-94).

The term "Bcl-2 inhibitors" as used herein refers to agents which inhibit the Bcl-2 protein interaction activity either by the inhibition of the phosphorylation of Bcl-2 ("Bcl-2 protein phosphorylation inhibitors") such as e.g., RTA-402 or by binding to the Bcl-2 protein and thus disruption of the Bad/Bcl-2 complex (these are referred to as "Bcl-2 protein binding inhibitors"). Preferably said Bcl-2 inhibitors are Bcl-2 protein binding inhibitors. The Bcl-2 inhibitory activity via direct binding of such Bcl-2 protein binding inhibitors can be measured via a competitive binding assay. Thus the IC50 of the inhibition of the Bcl-2 protein activity can be determined in an homogenous time resolved fluorescence (HTRF) Assay. Preferably the IC50 of anti-Bcl-2 inhibitory activity is 5 µM or less, more preferably 1 µM or less. Such Bcl-2 protein binding inhibitors include compounds such as Gossypol, AT-101, ABT-199 (also known as Venetoclax), Obatoclax mesylate, A-371191, A-385358, A-438744, ABT-737, ABT-263, AT-101, BL-11, BL-193, GX-15-003, 2-Methoxyantimycin A3, HA-14-1, KF-67544, Purpurogallin, TP-TW-37, YC-137 and Z-24.

"RTA-402" as used herein means CDDO-Me, the methyl ester of the C28-triterpenoid: oleanane triterpenoid 2-cyano-3,12-dioxoolean-1,9-dien-28-oic acid (CDDO) (See e.g., Honda, T., Rounds B V Bore, L., et al. J Med Chem. 43 (2000) 4233-4246), which blocks Bcl-2 protein phosphorylation (Konopleva, M., et al., Blood 99 (2002) 326-35).

Venetoclax (CAS Reg. No. 1257044-40-8; ABT-199; GDC-0199; RG-7601, AbbVie Inc., Genentech Inc.) is a BH3-mimetic drug designed to block the function of the protein Bcl 2 and is in Phase 3 clinical trials for the treatment of Multiple myeloma, Chronic lymphocytic leukemia, Systemic lupus erythematosus, Diffuse large B-cell lymphoma, Acute myelogenous leukemia, and Non-Hodgkin lymphoma (Souers et al Nat Med. 2013 Jan. 6. doi: 10.1038/nm.3048). Venetoclax has the IUPAC name: 4-(4-{[2-(4-chlorophenyl)-4,4-dimethylcyclohex-1-en-1-yl]methyl}piperazin- -1-yl)-N-({3-nitro-4-[(tetrahydro-2H-pyran-4-ylmethyl)amino]phenyl}sulfony- 1)-2-(1H-pyrrolo[2,3-b]pyridin-5-yloxy)benzamide.

ABT-737 as used herein means N-[4-[4-(4'-Chlorobiphenyl-2-ylmethyl)piperazin-1-yl]benzoyl]-3-[3-(dimethylamino)-1(R)-(phenylsulfanylmethyl)propylamino]-4-nitrobenzenesulfonamide; 4-[4-(4'-Chlorobiphenyl-2-ylmethyl)piperazin-1-yl]-N-[3-[3-(dimethylamino)-1(R)-(phenylsulfanylmethyl)propylamino]-4-nitrophenylsulfonyl]benzamide, a Bcl-2 inhibitor of formula I, which is described in WO 2006/099667 or Corey, S., et al., Cancer Cell 8 (2005) 5-6.

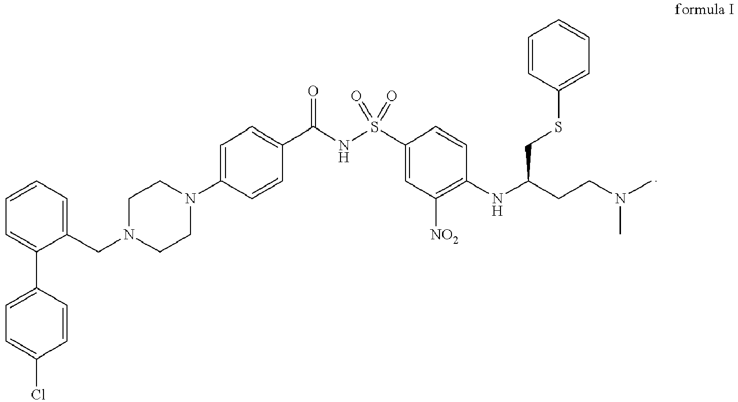

ABT-263 as used herein means a Bcl-2 inhibitor of formula II, which is described in US 2007/027,135,

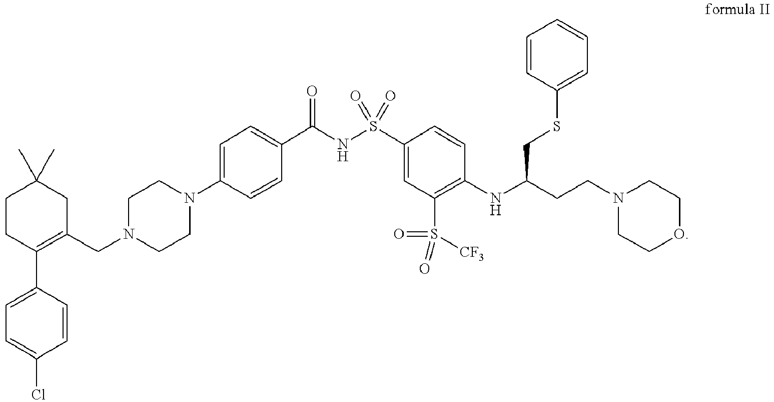

A-371191 as used herein means a Bcl-2 inhibitor of formula III,

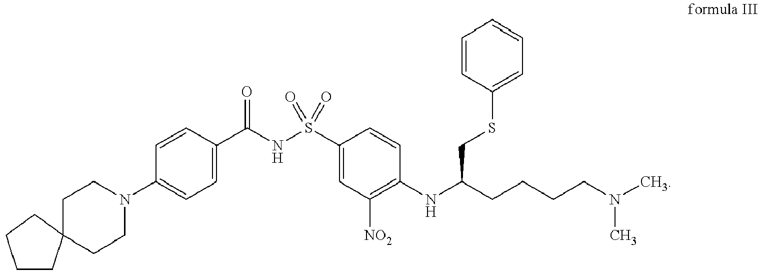

A-385358 as used herein means [(R)-4-(3-dimethylamino-1-phenylsulfanylmethyl-propylamino)-N-[4-(4,4-dimethyl-piperidin-1-yl)-benzoyl]-3-nitrobenzene-sulfonamide (as e.g., disclosed in Shoemaker, A. R., et al., Cancer Research 66 (2006) 8731-8739) a Bcl-2 inhibitor of formula IV,

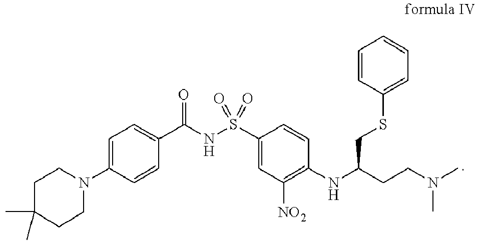

Gossypol as used herein means either a racemic mixture of (+)-Gossypol or (-)-Gossypol (a Bcl-2 inhibitor of formula V), or pure (+)-Gossypol or (-)-Gossypol, preferably Gossypol refers to pure (-)-Gossypol.

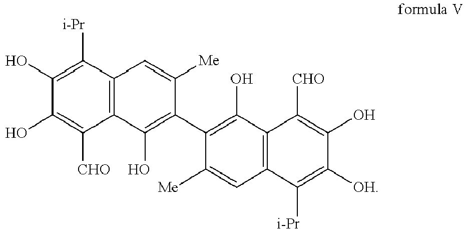

AT-101 as used herein means clinical lead compound of Ascenta Therapeutics AT-101, a Bcl-2 inhibitor and derivative of R(-)-gossypol.

Obatoclax mesylate (or other synonyms: GX-015-070;or GX15-070) as used herein means 2-[2-(3,5-Dimethyl-1H-pyrrol-2-ylmethylene)-3-methoxy-2H-pyrrol-5-yl]-1H-indole methanesulfonate, a Bcl-2 inhibitor, which is described e.g., in WO 2004/106328, WO 2006/089397 and Walensky, L. D., Cell Death and Differentiation, 13 (2006) 1339-1350. TW-37 as used herein means a Bcl-2 inhibitor of formula VI,

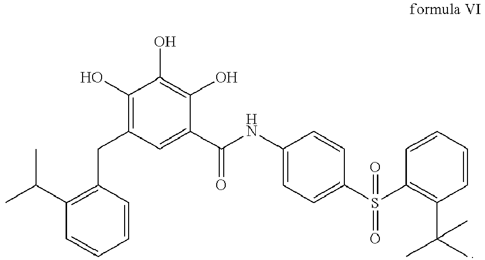

BL-193 as used herein means a Bcl-2 inhibitor of formula VII,

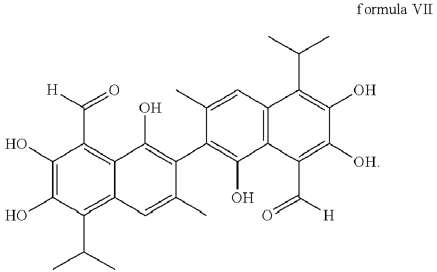

NSC-719664 as used herein means 2-Methoxy-Antimycin A3, a Bcl-2 inhibitor derived from Antimycin A₃.

YC-137 is described e.g., in Walensky, L. D., Cell Death and Differentiation 13 (2006) 1339-1350.

Purpurogallin is described e.g., in Walensky, L. D., Cell Death and Differentiation 13 (2006) 1339-1350.

HA-14-1 is described e.g., in Walensky, L. D., Cell Death and Differentiation 13 (2006) 1339-1350.

Z-24 as used herein means 3Z-3-[(1H-pyrrol-2-yl)-methylidene]-1-(1-piperidinylmethyl)-1,3-2H-indol-2-one, a Bcl-2 inhibitor of formula VIII,

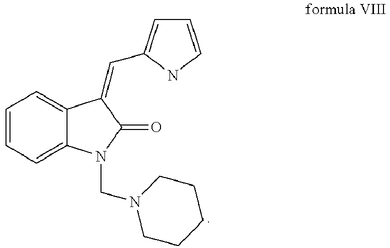

As mentioned, a therapeutically effective amount of a CXCR4 binding agent increases the expression of miR-15a, thereby treating the cancer

CXCR4 binding agents according to the present invention are compounds of SEQ ID NO: 14, such as BL8040.

Methods of analyzing the expression level of miR-15a or miR-16-1 are known in the art and include direct analysis of the expression level of these miRNAs or analyzing expression of their target genes, including for example Bcl-2, as described herein above.

The inhibitory agent can be administered immediately prior to (or after) the CXCR4 binding agent, on the same day as, one day before (or after), one week before (or after), one month before (or after), or two months before (or after) the described compounds, and the like.

The inhibitory agents (as described herein above) and the CXCR4 binding agents can be administered concomitantly, that is, where the administering for each of these reagents can occur at time intervals that partially or fully overlap each other. The inhibitory agents described herein and the CXCR4 binding agents can be administered during time intervals that do not overlap each other. For example, the inhibitory agent can be administered within the time frame of t=0 to 1 hours, while the CXCR4 binding agent can be administered within the time frame of t=1 to 2 hours. Also, the inhibitory agent can be administered within the time frame of t=0 to 1 hours, while the CXCR4 binding agents can be administered somewhere within the time frame of t=2-3 hours, t=3-4 hours, t=4-5 hours, t=5-6 hours, t=6-7 hours, t=7-8 hours, t=8-9 hours, t=9-10 hours, and the like. Moreover, the CXCR4 binding agents can be administered somewhere in the time frame of t=minus 2-3 hours, t=minus 3-4 hours, t=minus 4-5 hours, t=5-6 minus hours, t=minus 6-7 hours, t=minus 7-8 hours, t=minus 8-9 hours, t=minus 9-10 hours.

The CXCR4 binding agents and the inhibitory agents are typically provided in combined amounts to achieve therapeutic effectiveness. This amount will evidently depend upon the particular compound selected for use, the nature and number of the other treatment modality, the condition(s) to be treated, prevented and/or palliated, the species, age, sex, weight, health and prognosis of the subject, the mode of administration, effectiveness of targeting, residence time, mode of clearance, type and severity of side effects of the pharmaceutical composition and upon many other factors which will be evident to those of skill in the art.

In one exemplary embodiment, the amount of CXCR4 binding agent is used in an amount below the minimum dose required for therapeutic effectiveness when used as a single therapy (e.g., 10-99%, preferably 25 to 75% of that minimum dose). This allows for reduction of the side effects caused by the CXCR4 binding agent but the therapy is rendered effective because in combination with the inhibitory agents disclosed herein, the combinations are effective overall.

In another exemplary embodiment, the amount of the inhibitory agent is used in an amount below the minimum dose required for therapeutic effectiveness when used as a single therapy (e.g., 10-99%, preferably 25 to 75% of that minimum dose). This allows for reduction of the side effects caused by the inhibitory agent but the therapy is rendered effective because in combination with the CXCR4 binding agents disclosed herein, the combinations are effective overall.

In one embodiment, the inhibitory agents and the CXCR4 binding agents are synergistic with respect to their dosages. That is to say that the effect provided by the combination is greater than would be anticipated from the additive effects of the two agents when used separately. In an alternative but equally preferred embodiment, the inhibitory agents and the CXCR4 binding agents are synergistic with respect to their side effects. That is to say that the side-effects caused by the combination of the two agents are less than would be anticipated when the equivalent therapeutic effect is provided by either the agents when used separately.

A BCl-2 antagonist resistant subject is one that is less sensitive to the anticancer effects of BCl-2 antagonist than a non-resistant subject.

According to one embodiment, the BCl-2 antagonist resistant subject is insensitive to the anticancer effects of the BCl-2 antagonist.

According to one embodiment, the therapeutic effect of the BCl-2 antagonist is reduced by at least 10 %, 20 %, 30 %, 40 %, 50 %, 60 %, 70 %, 80 %, 90 % or more in the BCl-2 antagonist resistant subject as compared to the effect in a subject that does not have resistance (e.g., a subject just starting BCl-2 antagonist therapy).

It will be appreciated that the BCl-2 antagonist resistant subject may be more resistant to the effects of a particular BCl-2 antagonist (e.g., ABT199) than other BCl-2 antagonists.

As mentioned herein above, the present inventors have shown that CXCR4 binding agents increase miR-15a and 16-1, thereby reducing the target gene Bcl-2.

According to a particular embodiment, the cancers are neuroblastoma, retinoblastoma bladder cancer, esophageal carcinoma, sarcomas, colorectal cancer, melanoma, parathyroid adenoma, and breast cancer.

According to another embodiment, the cancer is a solid tumor.

As used herein the term "about" refers to ± 10 %.

The terms "comprises", "comprising", "includes", "including", "having" and their conjugates mean "including but not limited to".

The term "consisting of" means "including and limited to".

The term "consisting essentially of" means that the composition, method or structure may include additional ingredients, steps and/or parts, but only if the additional ingredients, steps and/or parts do not materially alter the basic and novel characteristics of the claimed composition, method or structure.

As used herein, the singular form "a", "an" and "the" include plural references unless the context clearly dictates otherwise. For example, the term "a compound" or "at least one compound" may include a plurality of compounds, including mixtures thereof.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

As used herein, the term "treating" includes abrogating, substantially inhibiting, slowing or reversing the progression of a condition, substantially ameliorating clinical or aesthetical symptoms of a condition or substantially preventing the appearance of clinical or aesthetical symptoms of a condition.

Various embodiments and aspects of the present invention as delineated hereinabove and as claimed in the claims section below find experimental support in the following examples.

### EXAMPLES

Reference is now made to the following examples, which together with the above descriptions illustrate some embodiments of the invention in a non limiting fashion.

Generally, the nomenclature used herein and the laboratory procedures utilized in the present invention include molecular, biochemical, microbiological and recombinant DNA techniques. Such techniques are thoroughly explained in the literature. See, for example, "Molecular Cloning: A laboratory Manual" Sambrook et al., (1989); "Current Protocols in Molecular Biology" Volumes I-III Ausubel, R. M., ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988); Watson et al., "Recombinant DNA", Scientific American Books, New York; Birren et al. (eds) "Genome Analysis: A Laboratory Manual Series", Vols. 1-4, Cold Spring Harbor Laboratory Press, New York (1998); methodologies as set forth in U.S. Pat. Nos. 4,666,828; 4,683,202; 4,801,531; 5,192,659 and 5,272,057; "Cell Biology: A Laboratory Handbook", Volumes I-III Cellis, J. E., ed. (1994); "Culture of Animal Cells - A Manual of Basic Technique" by Freshney, Wiley-Liss, N. Y. (1994), Third Edition; "Current Protocols in Immunology" Volumes I-III Coligan J. E., ed. (1994); Stites et al. (eds), "Basic and Clinical Immunology" (8th Edition), Appleton & Lange, Norwalk, CT (1994); Mishell and Shiigi (eds), "Selected Methods in Cellular Immunology", W. H. Freeman and Co., New York (1980); available immunoassays are extensively described in the patent and scientific literature, see, for example, U.S. Pat. Nos. 3,791,932; 3,839,153; 3,850,752; 3,850,578; 3,853,987; 3,867,517; 3,879,262; 3,901,654; 3,935,074; 3,984,533; 3,996,345; 4,034,074; 4,098,876; 4,879,219; 5,011,771 and 5,281,521; "Oligonucleotide Synthesis" Gait, M. J., ed. (1984); "Nucleic Acid Hybridization" Hames, B. D., and Higgins S. J., eds. (1985); "Transcription and Translation" Hames, B. D., and Higgins S. J., eds. (1984); "Animal Cell Culture" Freshney, R. I., ed. (1986); "Immobilized Cells and Enzymes" IRL Press, (1986); "A Practical Guide to Molecular Cloning" Perbal, B., (1984) and "Methods in Enzymology" Vol. 1-317, Academic Press; "PCR Protocols: A Guide To Methods And Applications", Academic Press, San Diego, CA (1990); Marshak et al., "Strategies for Protein Purification and Characterization - A Laboratory Course Manual" CSHL Press (1996).

Other general references are provided throughout this document.

### EXAMPLE 1

### Overexpression of CXCR4 leads to up regulation of mir15a and mir16-1

CXCR4 is overexpressed in the majority of tumors and its expression often correlates with bad prognosis. However, in the majority of tumor cell lines grown in culture, CXCR4 is expressed on the cell surface at low levels. It was found that overexpression of CXCR4 in various tumor cells increases their tumorigenicity in vitro and in vivo by increasing their survival signals through the ERK (FIG. 1A) signaling pathways. This finding is supported by numerous publications reporting that CXCL12/CXCR4 axis and its downstream signals PI3K/AKT PErk1/2 pathways play an important role in tumor progression and metastasis.

Unexpectedly, it was now found that over expressing CXCR4 in tumor cells leads to up regulation of mir15 and 16 (FIG. 1B). MicroRNAs encoded by the mir-15a/16-1 locus (mir-15a and mir-16-1-1) function as tumor suppressors. Expression of these miRNAs are downregulated in CLL, melanoma, colorectal cancer, bladder cancer and other solid tumors. mir-15a/16 cluster targets multiple oncogenes, including BCL2, and others. Indeed, up regulation of CXCR4 and mir15/16 lead to down regulation of mir15/16 target oncogenes mRNA; BCL-2, in a cell specific manner (FIG. 1C).

In the SKNBE neuroblastoma cells, overexpression of CXCR4 strongly upregulated mir15/16 and significantly downregulated BCL2 mRNA expression levels. Downregulation of BCL2 protein levels was also evident by Western Blot as well by internal FACS analysis (FIG. 1D). In correlation with the mRNA levels, reduced levels of BCL2 were observed in the PC3 cells but not RPMI-8226 cells (FIG. 1D).

Treatment of SKNBE with the CXCR4 binding agent BL8040 inhibited ERK signaling as well as induced mir-15a expression and reduced BCL-2 expression levels in these cells (FIG. 2D). The inhibition of these survival signals by BL8040 induces SKNBE cell death. Indeed up-regulation of mir-15a by transfection of cells with mir-15a or mir-16-1-1 induced SKNBE cell death (FIG. 2E).

To study the cross talk between the mir-15a/BCL2 axis, the AML cell line MV4-11 was analyzed. It was found that these cells are dependent on BCL-2 for their survival in vitro and can be induced to apoptosis using the ABT-199 at IC50 of 10nM (FIG. 3A). The CXCR4 binding agent BL8040 induced rapid and dose dependent apoptosis of MV4-11 cells inhibited ERK signaling as well as induced mir-15a expression and reduced significantly BCL2 expression levels (FIG. 3C). Furthermore, transfection of these cells with mir-15a or mir-16-1-1 also induces their cell death (FIG. 3D).

These results suggest that the CXCR4 binding agent BL8040 inhibit ERK signaling through the CXCR4 receptor, up regulate mir-15a/16-1 which in turn further deprived the cells from survival signals by down regulating the oncogenes BCL2.

B-cell chronic lymphocytic leukemia (CLL) is the most common adult leukemia. The most common chromosomal abnormalities detectable by cytogenetics include deletion at 13q. In 2002, it was discovered that a microRNA cluster mir-15a/mir-16-1-1 (mir-15a/16) is the target of 13q deletions in CLL. mir-15a/16 cluster targets multiple oncogenes, including BCL2 and others. The most important target of mir-15a/16 in CLL is arguably BCL2, as BCL2 is overexpressed in almost all CLLs. It was found that in CLL cells that are deleted from mir-15a/16 (CLL patient 1), BL8040 was unable to induce cell death (FIG. 3D). This suggests a critical role for this pathway in the induction of tumor cell death by this anti CXCR4 antagonist BL8040 (FIG. 3E).

To further evaluate the effect of BL8040 on tumor growth mir-15a/16-1 and BCL2 in vivo expression, neuroblastoma and leukemia mouse models in NSG mice were established using the SKNBE and MV4-11 cells. In the vivo orthotopic neuroblastoma (NB) model in NSG mice 0.5×10⁶ SKNBE cells were injected into the mice adrenal. One week later BL-8040 was injected daily for 3 weeks at 400ug/mouse treatment with BL-8040 induced tumor cell death and significantly inhibits tumor growth (FIG. 4A). In association, BL8040 treatment significantly increase mir15 and down regulated BCL2 in vivo (FIG. 4A).

In the in-vivo AML model with MV4-11 cells, 1×10⁶ cells were IV injected into NSG mice. Three week later BL-8040 was SC injected daily once (BL-8040x1) or twice (BL-8040x2) at 400ug/mouse. 24 hr after the last injection mice were sacrificed. BL8040 was found to induce AML apoptosis and reduce dramatically the number of AML cells in the spleen as demonstrated by FACS analysis following staining with human CD45 fluorescence antibody. Reduction in AML cells in the spleen was associated with a significant increase in miR15/16 and down regulation of BCL2 in human cells (FIG. 4B).

## Claims

1. A therapeutically effective amount of SEQ ID NO: 14 for use in rendering a BCl-2 antagonist resistant subject more susceptible to treatment with a BCl-2 antagonist, wherein said subject is suffering from cancer and wherein SEQ ID NO: 14 increases the expression of miR-15a in the cells of said cancer and wherein said BCL-2 antagonist is selected from the group consisting of Oblimersen, SPC-2996, RTA-402, Gossypol, AT-101, Obatoclax mesylate, A-371191, A-385358, A-438744, ABT-737, ABT-199, AT-101, BL-11, BL-193, GX-15-003, 2-Methoxyantimycin A3, HA-14-1, KF-67544, Purpurogallin, TP-TW-37, YC-137 and Z-24.

2. The therapeutically effective amount of SEQ ID NO: 14 for use of claim 1, wherein said BCl-2 antagonist is ABT-199.

3. The therapeutically effective amount of SEQ ID NO: 14 for use of any one of claims 1 or 2, wherein said cancer is selected from the group consisting of a hematological cancer, neuroblastoma, retinoblastoma, bladder cancer, esophageal carcinoma, sarcomas, colorectal cancer, melanoma, parathyroid adenoma and breast cancer.

4. The therapeutically effective amount of SEQ ID NO: 14 for use of any one of claims 1-3, wherein said cancer is a hematological cancer.

5. The therapeutically effective amount of SEQ ID NO: 14 for use of any one of claims 1-3, wherein said cancer is chronic lymphocytic leukemia (CLL).

6. The therapeutically effective amount of SEQ ID NO: 14 for use of any one of claims 1-3, wherein said cancer is neuroblastoma.

## Patentansprüche

1. Therapeutisch wirksame Menge von SEQ ID NO:14 zur Verwendung, um ein gegen BCL-2-Antagonisten resistentes Subjekt empfänglicher für die Behandlung mit einem BCL-2-Antagonisten zu machen, wobei das Subjekt an Krebs leidet und wobei SEQ ID NO: 14 die Expression von miR-15a in den Zellen des Krebses erhöht und wobei der BCL-2-Antagonist ausgewählt ist aus der Gruppe bestehend aus Oblimersen, SPC-2996, RTA-402, Gossypol, AT-101, Obatoclaxmesylat, A-371191, A-385358, A-438744, ABT-737, ABT-199, AT-101, BL-11, BL- 193, GX-15-003, 2-Methoxyantimycin A3, HA-14-1, KF-67544, Purpurogallin, TP-TW-37, YC-137 und Z-24.

2. Therapeutisch wirksame Menge von SEQ ID NO:14 zur Verwendung nach Anspruch 1, wobei der BCl-2-Antagonist ABT-199 ist.

3. Therapeutisch wirksame Menge von SEQ ID NO:14 zur Verwendung nach einem der Ansprüche 1 oder 2, wobei der Krebs ausgewählt ist aus der Gruppe bestehend aus hämatologischem Krebs, Neuroblastom, Retinoblastom, Blasenkrebs, Ösophaguskarzinom, Sarkomen, kolorektalem Krebs, Melanom, Nebenschilddrüsenadenom und Brustkrebs.

4. Therapeutisch wirksame Menge von SEQ ID NO:14 zur Verwendung nach einem der Ansprüche 1-3, wobei der Krebs ein hämatologischer Krebs ist.

5. Therapeutisch wirksame Menge von SEQ ID NO:14 zur Verwendung nach einem der Ansprüche 1-3, wobei der Krebs chronische lymphatische Leukämie (CLL) ist.

6. Therapeutisch wirksame Menge von SEQ ID NO:14 zur Verwendung nach einem der Ansprüche 1 bis 3, wobei der Krebs ein Neuroblastom ist.

## Revendications

1. Quantité thérapeutiquement efficace de SEQ ID NO : 14 pour son utilisation pour rendre un sujet résistant à un antagoniste de BCl-2 plus susceptible à un traitement avec un antagoniste de BCl-2, dans laquelle ledit sujet souffre d'un cancer et dans laquelle SEQ ID NO : 14 augmente l'expression de miR-15A dans les cellules dudit cancer et dans laquelle ledit antagoniste de BCL-2 est sélectionné dans le groupe constitué de l'oblimersen, de SPC-2996, de RTA-402, du gossypol, d'AT-101, du mésylate d'obatoclax, d'A-371191, d'A-385358, d'A-438744, d'ABT-737, d'ABT-199, d'AT-101, de BL-11, de BL-193, de GX-15-003, de la 2-méthoxyantimycine A3, d'HA-14-1, de KF-67544, de la purpurogalline, de TP-TW-37, d'YC-137 et de Z-24.

2. Quantité thérapeutiquement efficace de SEQ ID NO : 14 pour son utilisation selon la revendication 1, dans laquelle ledit antagoniste de BCl-2 est ABT-199.

3. Quantité thérapeutiquement efficace de SEQ ID NO : 14 pour son utilisation selon l'une quelconque des revendications 1 ou 2, dans laquelle ledit cancer est sélectionné dans le groupe constitué d'un cancer hématologique, d'un neuroblastome, d'un rétinoblastome, d'un cancer de la vessie, d'un carcinome de l'œsophage, de sarcomes, d'un cancer colorectal, d'un mélanome, d'un adénome parathyroïdien et d'un cancer du sein.

4. Quantité thérapeutiquement efficace de SEQ ID NO : 14 pour son utilisation selon l'une quelconque des revendications 1 à 3, dans laquelle ledit cancer est un cancer hématologique.

5. Quantité thérapeutiquement efficace de SEQ ID NO : 14 pour son utilisation selon l'une quelconque des revendications 1 à 3, dans laquelle ledit cancer est une leucémie lymphocytaire chronique (CLL).

6. Quantité thérapeutiquement efficace de SEQ ID NO : 14 pour son utilisation selon l'une quelconque des revendications 1 à 3, dans laquelle ledit cancer est un neuroblastome.
